# EUROPEAN PATENT APPLICATION

(11) **EP 2 827 567 A1**
(43) Date of publication of application: **21.01.2015**
(21) Application number: 12876830.6
(22) Date of filing: 11.06.2012
(51) Int. Cl.: H04M 1/60

(54) **SYSTEM AND METHOD FOR REDUCING DAMAGE TO AUDITORY ORGAN OF HUMAN BODY FROM TERMINAL SOUND PRESSURE**

(30) Priority: 15.05.2012 CN 201210150628
(71) Applicant: ZTE Corporation, Shenzhen, Guangdong 518057 (CN)
(72) Inventor: YAN, Bei, Shenzhen Guangdong 518057 (CN)
(74) Representative: Modiano, Micaela Nadia
(86) International application number: PCT/CN2012/076702
(87) International publication number: WO 2013/170515

(57) **Abstract**

A system and method for reducing damages of the terminal acoustic pressure to the human auditory organ is provided. The system includes: a temperature sensor module used for sensing a temperature within a preset distance and converting the temperature into an electrical signal; a terminal baseband processing module for converting the electrical signal into a temperature value, and notifying a sound loudness control module to reduce the sound loudness of the terminal when the temperature value rises from a temperature lower than the temperature of human ear to a temperature within the temperature range of human ear; and notifying the sound loudness control module to increase the sound loudness of the terminal when the temperature value drops from a temperature within the temperature range of human ear to a temperature lower than the temperature of human ear; a sound loudness control module for receiving the notification of reducing the sound loudness of the terminal sent by the terminal baseband processing module and reducing the sound loudness of the terminal; as well as receiving the notification of increasing the sound loudness of the terminal send by the terminal baseband module, and resuming the sound loudness of the terminal.

## Description

### Technical Field

The present invention relates to safety issue of a terminal acoustic pressure, and more particularly, to a system and method for reducing damage of the terminal acoustic pressure to the human auditory organ.

### Background of the Invention

Damage of terminal acoustic shock to human auditory organ is very common: children imitating adults to put a cell phone playing ring tones close to their ears (according to the current international standard for the acoustic shock, it is defined that users can feel the damage of acoustic shock when the distance from the speaker sound hole to the ear reference point (ERP) is less than or equal to 8cm), if the loudness of the playing ring tones causes excessive sound pressure level (more than 140dBSPL in the international standard), it would damage the children's auditory organs; a terminal user mistakenly places a terminal in the hands-free call close to his/her ear, it might also damage his/her auditory organ due to the excessive loudness of the acoustic signals played by the speaker.

With more and more concerns about the impact of the terminal acoustic shock upon human auditory organs, the field of terminal audio research & development and test have also begun to take the acoustic shock as an audio indicator for determining whether the acoustic signals of the terminal playing through the speaker damage the human auditory organ or not.

Currently, the technical solution applied in the industry to reduce the damage of the terminal acoustic shock to the human auditory organ is to reduce the Automatic Gain Control (AGC) in the entire audio channel, meanwhile reduce the loudness of the sound (such as the loudness of ringtone and the loudness of the sound of the hands-free call) played by the terminal player, so as to achieve the target that maximum sound pressure level (SPL) of the acoustic signals of the terminal playing through the speaker drops lower than 140 dBSPL.

While this method can reduce the damage of the terminal acoustic shock to the human auditory organ, once the loudness of the sound played by the terminal speaker is lowered, the loudness of the sound cannot be resumed, and after the human is far away from the terminal, the sound of the terminal cannot easily be heard, which is not conductive to the user experience.

### Summary of the Invention

The embodiment of the present invention provides a system and method for reducing damages of terminal acoustic pressure to human auditory organ, to solve the technical problem about how to flexibly adjust the terminal acoustic pressure, while ensure that the terminal acoustic pressure will not damage the human auditory organ.

The system for reducing the damage of terminal acoustic pressure to human auditory organ provided in an embodiment of the present invention comprises a temperature sensor module, a terminal baseband processing module and a sound loudness control module, wherein
said temperature sensor module is configured to: sense a temperature within a preset distance, and convert said temperature to an electrical signal;
said terminal baseband processing module is configured to: convert said electrical signal to a temperature value, and when determining that said temperature value rises from a temperature lower than a temperature of human auditory organ to a temperature within a temperature range of the human auditory organ, notify said sound loudness control module to reduce the sound loudness of terminal; and when determining that said temperature value decreases from a temperature within the temperature range of the human auditory organ to a temperature lower than the temperature of the human auditory organ, notify said sound loudness control module to increase the sound loudness of terminal;
said sound loudness control module is configured to: after receiving a notification of reducing the sound loudness sent from said terminal baseband processing module, reduce the sound loudness of said terminal; and after receiving a notification of increasing the sound loudness of said terminal sent from said terminal baseband module, resume the sound loudness of said terminal.

Alternatively, said sound loudness control module is configured to reduce the sound loudness of said terminal in the following way:
after receiving the notification of reducing the sound loudness sent from said terminal baseband processing module, according to the volume level of the sound loudness of said terminal and a corresponding relationship between a value of sound pressure level and the volume level, determining a volume level number required to reduce the value of sound pressure level at least to 140dBSPL, wherein said volume level number is taken as a value that is at least required to be reduced in the sound loudness of said terminal.

Alternatively, said temperature sensor module is configured to sense the temperature in a preset distance in the following manner:
sensing the temperature within the preset distance at a frequency of 2HZ or greater than 2HZ.

Alternatively, said system further comprises a call mode control module,
said terminal baseband processing module is further configured to: determine whether the sound of said terminal is the sound of hands-free call or not, if determining that the sound of said terminal is the sound of hands-free call and a temperature value sensed by the temperature sensor module rises from a temperature lower than the temperature of human auditory organ to a temperature within the temperature range of human auditory organ, notify said call mode control module to switch a call mode from a hands-free state into a handhold state; if determining that the sound of said terminal is the sound of handhold call, and the temperature value sensed by the temperature sensor module reduces from a temperature within the temperature range of human auditory organ to a temperature lower than the temperature of human auditory organ, notify said call mode control module to resume the call mode;
said call mode control module is configured to: receive a notification from said terminal baseband processing module, and perform corresponding operations.

Alternatively, said temperature sensor module is used to sense temperatures within an adjustable preset distance.

The method for reducing damages of terminal acoustic pressure to human auditory organ provided in the present invention comprises:
sensing a temperature within a preset distance, and converting the temperature to an electrical signal;
converting said electrical signal to a temperature value;
   when the temperature value rises from a temperature lower than a temperature of human auditory organ to a temperature within a temperature range of the human auditory organ, reducing a sound loudness of a terminal; when the temperature decreases from a temperature within the temperature range of the human auditory organ to the temperature lower than the temperature of the human auditory organ, resume the sound loudness of the terminal.

Alternatively, said reducing the sound loudness of the terminal comprises:
according to a volume level of the sound loudness of the terminal and a corresponding relationship between a value of sound pressure level and the volume level, determining a volume level number required to reduce the value of sound pressure level at least to 140dBSPL, taking said volume level number as a value that is at least required to be reduced in the sound loudness of the terminal.

Alternatively, said sensing the temperature within the preset distance comprises:
sensing the temperature within said preset distance at a frequency of 2HZ or greater than 2HZ.

Alternatively, said method further comprises:
determine whether a sound of said terminal is a sound of hands-free call or not, if determining that the sound of said terminal is the sound of hands-free call and a sensed temperature value rises from a temperature lower than the temperature of human auditory organ to a temperature within the temperature range of the human auditory organ, switch a call mode from a hands-free state into a handhold state; if determining that the sound of said terminal is a sound of a handhold call, and the sensed temperature value drops from a temperature within the temperature range of the human auditory organ to a temperature lower than the temperature of the human auditory organ, resume the call mode.

Alternatively, said sensing the temperature within said preset distance comprises:
sensing the temperature within an adjustable preset distance.

By sensing the temperature, in the abovementioned embodiments, it is determined whether the human auditory organ is close to or away from the terminal speaker or not, and when the human auditory organ is close to the terminal speaker, reducing the sound loudness played out by the terminal speaker, and resuming the original sound loudness of said terminal when the human auditory organ is far away from the terminal speaker, which not only reduces the damage of the acoustic shock to the human auditory organ, but also ensures the user experience.

### Brief Description of the Drawings

FIG. 1 shows the compositions of a system for reducing the damage of terminal acoustic pressure to the human auditory organ in accordance with the present embodiment;
FIG. 2 is a schematic diagram of the position of the temperature sensor module in the terminal in accordance with the present embodiment;
FIG. 3 is a flow chart of the method for reducing the damage of the terminal acoustic pressure to the human auditory organ in accordance with the present embodiment.

### Preferred Embodiments of the Present Invention

Hereinafter the embodiments of the present invention will be described in detail with the combination of the accompanying drawings. It should be noted that, in the case of no conflict, the embodiments of the present application and the features in the embodiments might be combined with each other arbitrarily.

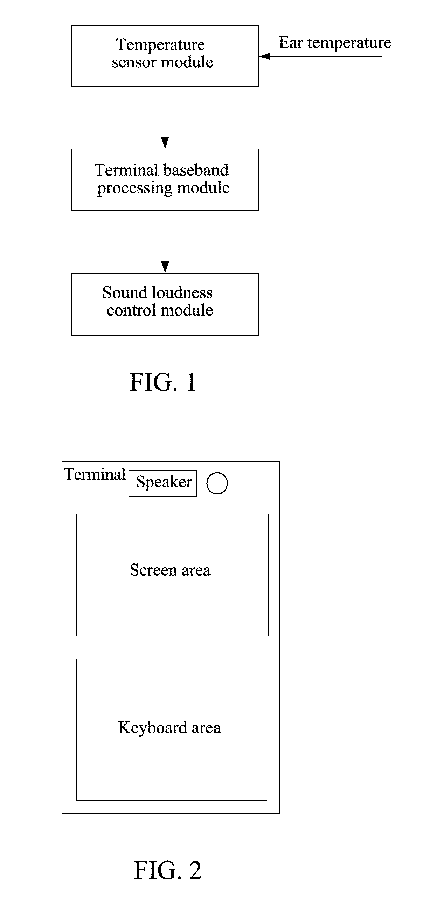

FIG. 1 shows the components of a system for reducing the damage of the terminal acoustic pressure to the human auditory organ in accordance with the present embodiment.

The system comprises a temperature sensor module, a terminal baseband processing module, and a sound loudness control module, wherein
said temperature sensor module is used to sense a temperature within a preset distance, and convert said temperature to an electrical signal;
said temperature sensor module is used to sense the temperature within said preset distance at a frequency of 2HZ or greater than 2HZ;
said temperature sensor module can be used to sense the body temperature by detecting the infrared spectrum emitted from the hypothalamus (equivalent to detecting the tympanic membrane), and convert the optical signal into an electrical signal;
said temperature sensor module can be set in a hole next to the terminal speaker, as shown in FIG. 2, and preferably, its position should be as close to the speaker as possible. For a terminal in which the earpiece and speaker are combined into one, it can be set in the hole next to the earpiece. The size of said hole can be determined according to the appearance structures of the terminal product and the size of the sensor probe built in said temperature sensor module;
the sensor built in the temperature sensor module can be an infrared sensor or a thermopile sensor. Depending on different types of the built-in sensors, the electrical signal output by the sensor can be a current signal or a voltage signal; if the electrical signal output by the sensor is a current signal, a current-to-voltage converter sub-module needs to be set in said temperature sensor module to convert said current signal into a voltage signal and output it to the terminal baseband processing module;

Furthermore, the temperature sensor module can be configured to sense the temperature within 8cm, that is, when the distance from the human body to the temperature sensor module is less than or equal to 8cm, the temperature sensor module is in a working state. Furthermore, the temperature sensor can be configured to sense the temperature within an adjustable preset distance as desired;
said terminal baseband processing module is used to convert said electrical signal into a temperature value, and when determining that said temperature value rises from a temperature lower than the temperature of human auditory organ to a temperature within the temperature range of the human auditory organ, notify the sound loudness control module to reduce the sound loudness of said terminal; and when determining that the temperature drops from a temperature within the temperature range of the human auditory organ to a temperature lower than the temperature of the human auditory organ, notify the sound loudness control module to increase the sound loudness of the terminal;
said sound loudness control module is used to: after receiving a notification of reducing the sound loudness sent from said terminal baseband processing module, reduce the sound loudness of the terminal; after receiving a notification of increasing the sound loudness of the terminal sent from said terminal baseband module, resume the sound loudness of the terminal;
said sound loudness control module calculates a value that is required to be reduced according to the sound loudness of the terminal after receiving the notification of reducing the sound loudness sent from said terminal baseband processing module: according to the volume level of the sound loudness of said terminal, and the corresponding relationship between a value of sound pressure level and the volume level, determining a volume level number required to reduce the value of sound pressure level at least to 140dBSPL, wherein said volume level number is taken as a value that is at least required to be reduced in the sound loudness of said terminal.

In other embodiments, the abovementioned system may further comprise a call mode control module, and said call mode control module can be used to receive the notification from said baseband processing module and perform the corresponding operations;
at this time, the terminal baseband processing module is further used to determine whether the sound of said terminal is the sound of hands-free call sound or not, if it determines that the sound of said terminal is the sound of hands-free call and the temperature value sensed by the temperature sensor module rises from a temperature lower than the temperature of human auditory organ to a temperature within the temperature range of the human auditory organ, notify the call mode control module to switch the call mode from a hands-free state to a handhold state; if the sound of said terminal is the sound of the handhold call, and the temperature value sensed by the temperature sensor module drops from a temperature within the temperature range of the human auditory organ to a temperature lower than the temperature of the human auditory organ, notify the call mode control module to resume the call mode.

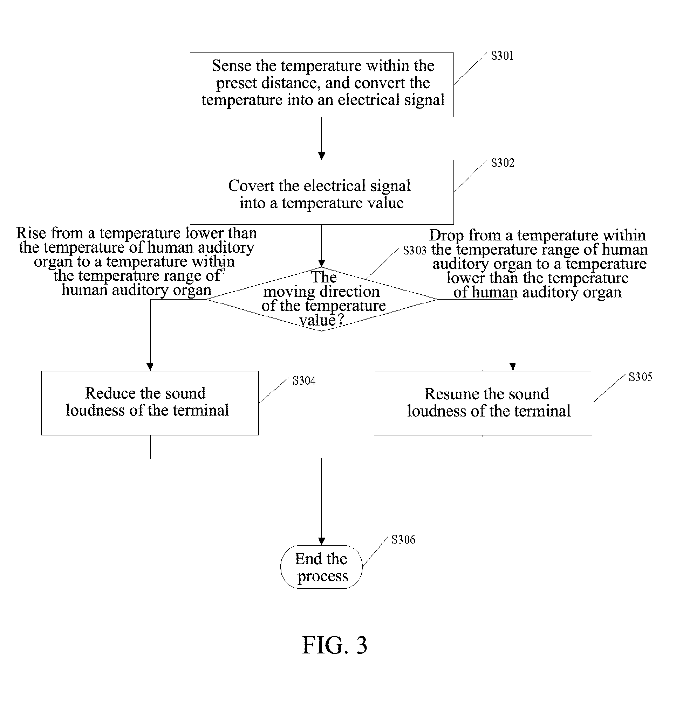

FIG. 3 is a flow chart of the method for reducing the damage of terminal acoustic pressure to human auditory organ in accordance with the present embodiment.

S301, the temperature is sensed within a preset distance, converting the temperature to an electrical signal;
taking into account the speed of a human hand moving to the ear, the temperature within the preset distance can be sensed at the frequency of 2HZ or greater than 2HZ;
the temperature within 8cm can be sensed, or the temperature can be sensed within an adjustable preset distance as desired;

S302, the electric signal is converted into a temperature value;

S303, the moving direction of said temperature value is determined, and if the temperature value rises from a temperature lower than the temperature of human auditory organ to a temperature within the temperature range of the human auditory organ, proceed to step S304; if the temperature value drops from a temperature within the temperature range of the human auditory organ to a temperature lower than the temperature of the human auditory organ, proceed to step S305;

S304, the sound loudness of said terminal is reduced, proceeding to step S306;
the method for reducing the sound loudness of the terminal can be: according to the volume level of the sound loudness of the terminal and the corresponding relationship between a value of sound pressure level and the volume level, determining a volume level number required to reduce the value of sound pressure level at least to 140dBSPL, taking said volume level number as a value that is at least required to be reduced in the sound loudness of said terminal; wherein, the corresponding relationship between the value of sound pressure level and the volume level can exist in the form of a table;

S305, the sound loudness of said terminal is resumed, proceeding to step S306;

S306, the process ends.

In other embodiments, in addition to determining the moving direction of the temperature value in the abovementioned step S303 the, it is also determined whether the sound of said terminal is the sound of hands-free call or not; if the sound of said terminal is determined as the sound of hands-free call, and the sensed temperature value rises from a temperature lower than the temperature of human auditory organ to a temperature within the temperature range of the human auditory organ, the call mode is switched from a hands-free state to a handhold state; if it is determined that the sound of said terminal is the sound of handhold call, the sensed temperature value drops from a temperature within the temperature range of the human auditory organ to a temperature lower than the temperature of the human auditory organ, the call mode is resumed.

Those ordinarily skilled in the art can understand that all or some of steps of the above-mentioned method may be completed by the programs instructing the relevant hardware, and said programs may be stored in a computer-readable storage medium, such as read only memory, magnetic or optical disk. Optionally, all or some of the steps of the abovementioned embodiments may also be implemented by using one or more integrated circuits. Accordingly, each module/unit in the abovementioned embodiment may be realized in the form of hardware or software function module. The present invention is not limited to any specific form of hardware and software combinations.

It should be noted that, the present invention has a variety of other embodiments, and without departing from the spirit and principle of the present invention, those skilled in the field can make a variety of changes and modifications according to the embodiments of the present invention, and these changes and modifications should be included within the protection scope of the appended claims of the present invention.

### Industrial Applicability

By sensing the temperature, in the abovementioned embodiments, it is determined whether the human auditory organ is close to or away from the terminal speaker or not, and when the human auditory organ is close to the terminal speaker, reducing the sound loudness played out by the terminal speaker, and resuming the original sound loudness of said terminal when the human auditory organ is far away from the terminal speaker, which not only reduces the damage of the acoustic shock to the human auditory organ, but also ensures the user experience.

## Claims

1. A system for reducing a damage of a terminal acoustic pressure to a human auditory organ, wherein, said system comprises a temperature sensor module, a terminal baseband processing module and a sound loudness control module, wherein
said temperature sensor module is configured to: sense a temperature within a preset distance, and convert said temperature to an electrical signal;
said terminal baseband processing module is configured to: convert said electrical signal to a temperature value, and when determining that said temperature value rises from a temperature lower than a temperature of human auditory organ to a temperature within a temperature range of the human auditory organ, notify said sound loudness control module to reduce a sound loudness of terminal; and when determining that said temperature value decreases from a temperature within the temperature range of the human auditory organ to a temperature lower than the temperature of the human auditory organ, notify said sound loudness control module to increase the sound loudness of the terminal;
said sound loudness control module is configured to: after receiving a notification of reducing the sound loudness sent from said terminal baseband processing module, reduce the sound loudness of the terminal; and after receiving a notification of increasing the sound loudness of the terminal sent from said terminal baseband module, resume the sound loudness of the terminal.

2. The system of claim 1, wherein, said sound loudness control module is configured to reduce the sound loudness of said terminal in the following way:
according to a volume level of the sound loudness of said terminal and a corresponding relationship between a value of sound pressure level and the volume level, determining a volume level number required to reduce the value of sound pressure level at least to 140dBSPL, taking said volume level number as a value that is at least required to be reduced in the sound loudness of said terminal.

3. The system of claim 1, wherein, said temperature sensor module is configured to sense a temperature in a preset distance in the following way:
sensing the temperature within said preset distance at a frequency of 2HZ or greater than 2HZ.

4. The system of any one of claims 1∼3, wherein, said system further comprises a call mode control module,
wherein said terminal baseband processing module is further configured to: determine whether a sound of said terminal is a sound of hands-free call or not, if determining that the sound of said terminal is a sound of hands-free call and a temperature value sensed by the temperature sensor module rises from a temperature lower than the temperature of the human auditory organ to a temperature within the temperature range of the human auditory organ, notify said call mode control module to switch a call mode from a hands-free state into a handhold state; if determining that the sound of said terminal is a sound of handhold call, and the temperature value sensed by the temperature sensor module reduces from a temperature within the temperature range of the human auditory organ to a temperature lower than the temperature of the human auditory organ, notify said call mode control module to resume the call mode;
said call mode control module is configured to: receive a notification from said terminal baseband processing module, and perform corresponding operations.

5. The system of claim 4, wherein,
said temperature sensor module is configured to sense the temperature within an adjustable preset distance.

6. A method for reducing a damage of a terminal acoustic pressure to a human auditory organ, said method comprising:
sensing a temperature within a preset distance, and converting the temperature to an electrical signal;
converting said electrical signal to a temperature value; and
when the temperature value rises from a temperature lower than a temperature of human auditory organ to a temperature within a temperature range of the human auditory organ, reducing a sound loudness of a terminal; when the temperature decreases from a temperature within the temperature range of the human auditory organ to the temperature lower than the temperature of the human auditory organ, resume the sound loudness of the terminal.

7. The method of claim 6, wherein, the step of reducing the sound loudness of said terminal comprises:
according to a volume level of the sound loudness of the terminal and a corresponding relationship between a value of sound pressure level and the volume level, determining a volume level number required to reduce the value of sound pressure level at least to 140dBSPL, taking said volume level number as a value that is at least required to be reduced in the sound loudness of the terminal.

8. The method of claim 6, wherein, the step of sensing the temperature within said preset distance comprises: sensing the temperature within said preset distance at a frequency of 2HZ or greater than 2HZ.

9. The method of any one of claims 6∼8, wherein, said method further comprises:
determining whether a sound of said terminal is a sound of hands-free call or not, if determining that the sound of said terminal is the sound of hands-free call and a sensed temperature value rises from a temperature lower than the temperature of the human auditory organ to a temperature within the temperature range of the human auditory organ, switching a call mode from a hands-free state into a handhold state; if determining that the sound of said terminal is a sound of a handhold call, and the sensed temperature value drops from a temperature within the temperature range of the human auditory organ to a temperature lower than the temperature of the human auditory organ, resuming the call mode.

10. The method of claim 9, wherein, the step of sensing the temperature within said preset distance comprises:
sensing the temperature within an adjustable preset distance
